# EUROPÄISCHE PATENTSCHRIFT

(11) **EP 0 768 868 B1**
(45) Veröffentlichungstag und Bekanntmachung des Hinweises auf die Patenterteilung: **29.08.2001**
(21) Anmeldenummer: 95922463.5
(22) Anmeldetag: 19.05.1995
(51) Int. Cl.: A61K 9/00

(54) **PHARMAZEUTISCHE ZUBEREITUNG MIT EINEM HYDROPHOBEN WIRKSTOFF UND EINEM BRAUSESYSTEM, SOWIE VERFAHREN ZUR HERSTELLUNG DER ZUBEREITUNG**
PHARMACEUTICAL PREPARATION WITH A HYDROPHOBIC ACTIVE SUBSTANCE AND AN EFFERVESCENT SYSTEM, AND PROCESS FOR PREPARING SAID PREPARATION
PREPARATION PHARMACEUTIQUE COMPORTANT UN PRINCIPE ACTIF HYDROPHOBE ET UN SYSTEME EFFERVESCENT, ET PROCEDE DE FABRICATION DE LADITE PREPARATION

(30) Priorität: 15.06.1994 CH 189094
(43) Veröffentlichungstag der Anmeldung: 23.04.1997
(73) Patentinhaber: Gergely, Gerhard, Dr., A-1053 Wien (AT)
(72) Erfinder: GERGELY, Gerhard, A-1053 Wien (AT); GERGELY, Irmgard, A-1053 Wien (AT); GERGELY, Thomas, A-1053 Wien (AT)
(74) Vertreter: Büchel, Kurt F., Dr.
(86) Internationale Anmeldenummer: EP9501904
(87) Internationale Veröffentlichungsnummer: WO9534284

(56) Entgegenhaltungen:
- EP-A- 0 228 164
- WO-A-94/10994

## Beschreibung

Die Erfindung betrifft eine pharmazeutische Zubereitung nach dem Oberbegriff des Anspruchs 1. Hydrophobe Wirkstoffe ergeben bei der galenischen Zubereitung mit einem Brausesystem beträchtliche Schwierigkeiten, die sich im Fall von Loratadin noch vermehren: abgesehen von der Bildung von Wirkstoffrändern am Glas wird auch die Auflösung einer Brausetablette wesentlich verlangsamt. Eine besondere Zusammensetzung des Brausesystems, wie sie beispielsweise für säure-, alkali- und/oder metallempfindliche Wirkstoffe hilfreich sein kann, hilft hier nicht weiter.

Beispielsweise ist Loratadin nahezu wasserunlöslich (2.5 mg/ Liter), und hat einen sehr stark hydrophoben Charakter. Es ist somit äusserst schlecht benetzbar und daher schlecht suspendierbar. Seine Feinteilchen haben ausserdem die Tendenz, an der Wasseroberfläche einen Film zu bilden, verstärkt an der Glaswand hinaufzukriechen und dort relativ stark zu haften; die gröberen Teilchen sinken zu Boden; zusätzlich ergibt sich beim Auflösen einer herkömmlichen, Loratadin enthaltenden Brausetablette eine Schaumbildung, womit sich ein für die Vermarktung nicht geeignetes Auflöseverhalten ergibt. Durch das Haften am Glas gehen übrigens ausserdem bis zu 10% des Wirkstoffes verloren.

Eine weitere Problematik besteht in der geringen Dosierung von 10 mg Wirkstoff pro Tablette, die in einer Brausetablette eine entsprechend den pharmazeutischen Richtlinien geforderte Content Uniformity erreichen muss. Auch dieser Anforderung kann durch Zumischen des Wirkstoffes zu einer Brausebasis nicht entsprochen werden. Angestrebt ist ausserdem eine kleine Brausetablette von ca. 1 bis 1.5 g, was die Problemlösung erschwert.

Um die schlechten Suspensionseigenschaften des Loratadins zu verbessern, wurde schon versucht, entsprechend der EP A1 241 469 den Wirkstoff mit Benetzungsmitteln wie Docusat-Natrium oder Natriumlaurylsulfat, mit und ohne Bindemittel, zu behandeln. Dabei zeigte sich, dass der Einsatz dieser Benetzungsmittel nicht zielführend war, und zwar weder durch direktes Aufbringen des Tensides auf den Wirkstoff noch auf eine Mischung des Wirkstoffs mit einem Füllstoff wie Mannit oder Sorbit, noch durch das Hinzufügen des Tensides zur Brausebasis. Der so behandelte Wirkstoff zeigte in der Brausetablette beim Auflösen zu starke Schaumbildung, die auch durch Zusatz eines Antischaummittels nicht wesentlich reduziert werden konnte, wobei weiters nachteilig beobachtet werden konnte, dass der Wirkstoff nicht suspendiert wird, sondern sich im Schaum an der Wasseroberfläche sammelte.

Die Erfindung hat sich nun die Aufgabe gestellt, eine galenische Zubereitung für hydrophobe Wirkstoffe, insbesondere für Loratadin, zu schaffen, die dem physikalischen Verhalten solcher Wirkstoffe Rechnung trägt. Dabei darf es aber auch zu keinen anderen unerwünschten Nebenerscheinungen kommen, wie zum Beispiel zu einer Schaumbildung. Des weiteren sollten auch geringe Wirkstoffmengen in einer Brausetablette möglichst gleichmässig verteilt werden können, und das Gewicht der Brausetablette sollte so klein wie möglich gehalten werden können.

Die Lösung dieser Aufgabenstellungen bzw. Probleme ergibt sich erstmalig überraschend durch die im Kennnzeichen des Anspruchs 1 beschriebenen Massnahmen; besondere Ausführungsformen und Weiterentwicklungen des Erfindungsgedankens sind in den Kennzeichen der abhängigen Ansprüche beschrieben. Die Erfindung wird zunächst am Beispiel Loratadin näher erläutert. Als "Tensid" im Sinne der Erfindung sind die in der Pharmazie üblichen, die Oberflächenspannung reduzierenden, insbesondere (aber nicht ausschliesslich) ionische Netzmittel zu verstehen, sowie Docusat-Natrium, Natriumlaurylsulfat etc. Besonders zweckmässig ist es, wenn die erfindungsgemässe Zubereitung neben einem derartigen "Tensid" wenigstens einen "Emulgator", oder statt dessen zwei "Emulgatoren" enthält. Als "Emulgatoren" sind die in der Pharmazie als übliche Hilfsmittel zur Erzielung von Wasser-in-Öl- oder Öl-in-Wasser-Emulsionen dienenden Netzmittel zu verstehen, insbesondere Zuckertenside, Phospholipide und Polysorbate.

Erfindungsgemäss werden des weiteren Wege vorgeschlagen, um die Schaumbildung hintanzuhalten, den Wirkstoff während des Auflösens der Brausetablette zu suspendieren und sein Haften am Glas zu verhindern. Dies gelingt durch die Massnahme der Herstellung eines eigenen Wirkstoffgranulates, das bevorzugt ein oder zwei verschiedene Emulgatoren in Kombination enthält und der Brausebasis zugefügt wird.

Durch die Massnahme der Beladung eines Schwebstoffes mit dem unlöslichen Wirkstoff ergibt sich jedoch noch immer ein geringer Nachteil, dass nämlich die Teilchen zu schwer werden und die Tendenz zeigen, beim Auflösen der Tablette zu Boden zu sinken. Weiters kann durch die oft nur geringe Dosierungsmenge dieser Stoffe auch noch nicht die geforderte Gleichverteilung des Wirkstoffes in der Brausetablette erreicht werden. Jedoch konnte auch dieses Problem gelöst werden, indem man den Wirkstoff auf einem zusätzlichen, nicht zu leicht löslichen Füllstoff verankert. Durch diese "Verdünnung" kann die Gleichverteilung in der Tablette erreicht, wie auch das Suspensionsverhalten, entscheidend verbessert werden, da der Wirkstoff sowohl auf dem Schwebstoff wie auf dem Füllstoff verankert ist. Es enthält somit das Wirkstoffgranulat einen Neutralstoff als Träger oder Füllstoff, sowie einen Schwebstoff, wie Aerosil^{(R)}, und ein Bindemittel, mit dem der Wirkstoff an den Träger bzw. Füllstoff gebunden wird.

Um daher hydrophobe Wirkstoffe - ohne ihr negatives Verhalten beim späteren Auflösen in Kauf nehmen zu müssen - in eine Brausetablette einarbeiten zu können, ist es besonders zweckmässig, spezielle Phasen zuzubereiten, z.B. in die Brausetablette eine wirkstoffspezifische, spezielle Kombination von Suspensionshilfsmitteln oder oberflächenaktiven Substanzen einzubringen und vorzugsweise zusätzlich eine Wirkstoff-Phase herzustellen. Die Herstellung einer Wirkstoffphase allein genügt aber erfahrungsgemäss nicht, um die unschönen Eigenschaften der Wirkstoffe beim Auflösen der Brausetablette hintanzuhalten.

Für die Herstellung des Wirkstoffgranulates gibt es zwei Möglichkeiten: Entweder man löst den Wirkstoff und zieht ihn auf einen Schwebstoff bzw. Antiklebstoff als Träger auf, der beim Auflösen der Brausetablette in Wasser suspendiert, wie z.B. Aerosil^{(R)} oder Avicel^{(R)} (mikrokristalline Zellulose); oder man mischt den Träger mit dem Wirkstoff und löst dann den Wirkstoff an, vorzugsweise mittels eines ein Bindemittel enthaltenden Lösungsmittels, damit er sich an den Träger anhängen kann. Wenn auch der Träger bzw Füllstoff von dem Lösungsmittel wenigstens angelöst wird, kann unter Umständen auf das Bindemittel verzichtet werden. Es wird also eine Wirkstoffphase hergestellt, bei der der Wirkstoff entweder angelöst oder aufgelöst wird, vorzugsweise zusammen mit einem Bindemittel, insbesondere Polyvinylpyrrolidon. Der Wirkstoff wird dabei an der Oberfläche des Schwebstoffes bzw. Antiklebestoffes mit dem Bindemittel kopräzipitiert und so beim Auflösen der Brausetablette weitgehendst in Schwebe gehalten. Wie bereits erwähnte wird jedoch der Wirkstoff bevorzugt an die Mischung eines Schwebstoffes mit einem Füllstoff wie Mannit, Lactose, Maltodextrin oder Saccharose als Träger "angebunden".

Der Träger kann entweder - zum Beispiel in einer Loratadin-Phase - aus Lactose plus Aerosil^{(R)} und Natriumbicarbonat bestehen, oder - wie z.B. beim Cisaprid, gegebenenfalls - aus Aerosil^{(R)} allein. Um aber diese Phase in der Brauselösung optimal zu suspendieren und die oben genannten negativen Eigenschaften zu kompensieren, ist es nötig, in der Gesamtrezeptur wenigstens zwei oberflächenaktive Substanzen zu verwenden, zum Beispiel bei Cisaprid: Docusat-Natrium und Metarin^{(R)}P (ein pulvriges, fremdfettarmes Phosphoaminolipid); bei Loratadin: Epikuron^{(R)} (ebenfalls ein Lecithin) und einen DK-Ester^{(R)} (ein Zucker-Fettsäureester), die alle dispergierende Eigenschaften haben.

Emulgatoren einerseits aus der Gruppe der Zuckerester (z.B. DK-Ester^{(R)}), Polysorbate (z.B. Tween^{(R)}), andererseits der Phospholipide (z.B. Lecithine, Phosphatidylcholin, Metarin^{(R)}, Epikuron^{(R)}, Phosphatidylethanolamin, Phosphatidylinosit etc.) können entweder in das Wirkstoffgranulat inkorporiert oder getrennt von diesem direkt auf das Brausegranulat oder auf einen Neutralstoff aufgezogen und anschliessend dem Wirkstoffgranulat und Brausegranulat zugefügt werden. Allerdings hat sich gezeigt, dass durch die Granulierung mit dem Bindemittel die Wirkung des Emulgators im Wirkstoffgranulat nicht ganz so gut ist, wie wenn er im Falle der Festform direkt der Endmischung zugefügt wird.

Insbesondere für die flüssigen oder pastösen Phospholipide wird bevorzugt ein Emulgatorgranulat getrennt von der Brausebasis und vom Wirkstoffgranulat hergestellt, wobei als Träger für den Emulgator fast alle für eine Brausetablette geeigneten Füllstoffe, wie höhere Alkohole, z.B. Mannit, Sorbit oder Lactose, oder Hydrokolloide, wie Maltodextrin oder Dextrine, oder auch Stärke in Frage kommen. Die Menge, auf die der Emulgator aufgezogen wird, ist unkritisch; sie sollte jedoch einerseits für die erforderliche Einarbeitung in eine Brausetablette genügen; andererseits sollte das Granulat so beschaffen sein, dass es nicht zu fettig ist. Beides verlangt zumindest ein Verhältnis von 1 Teil Emulgator auf 10 Teile Träger.

Bei der Emulgatorphase wird der Emulgator, z.B. das Phosphatidylcholin, mittels eines Lösungsmittels oder als wässrige Suspension auf einen Füllstoff aufgezogen, um innerhalb der Tablette eine möglichst ubiquitäre Verteilung zu erreichen.

Durch die bereits erwähnte, erfindungsgemässe Kombination zweier Tenside bzw. Emulgatoren, einerseits aus der Gruppe der Phospholipide, andererseits der Zuckerester (insbesondere von Speisefettsäuren) wird das Hinaufkriechen des Wirkstoffes an der Glaswand von ursprünglich mindestens 10% auf nunmehr maximal 2 - 3 % reduziert. Unter den Zuckerestern gibt es solche, die hydrophiler, und solche, die lipophiler sind. Interessant ist, dass beim Loratadin der DK-Ester^{(R)}F 50, der an sich ein lipophiler Zuckerester ist, bessere Ergebnisse bringt als die hydrophilen Zuckerester, z.B. DK-Ester^{(R)}F 140. Dies mag dadurch bewirkt sein, dass das Schaumvermögen dieses DK-Esters^{(R)} am geringsten ist.

### Beispiel 1:

10 Gewichtsteile Loratadin werden mit 150 Gewichtsteilen Mannit sowie 2.5 Gewichtsteilen Aerosil^{(R)} gemischt und unter Mischen auf 55°C erwärmt. 5 Gewichtsteile Polyvinylpyrrolidon und 0.5 Gewichtsteile eines Polysorbat-Emulgators (z.B. Tween^{(R)}) werden in 30 Gewichtsteilen Ethanol gelöst und unter Rühren auf die Mischung aufgebracht. Es wird 5 min zur gleichmässigen Benetzung gemischt; anschliessend wird das Granulat mittels Vakuum bei einer Temperatur von 50°C getrocknet. Das so hergestellte Wirkstoffgranulat wird auf 0.2 mm gesiebt und zu 1'000 Gewichtsteilen einer Brausebasis, der 2.4 Gewichtsteile DK-ester^{(R)}, sowie Aromen und Süßstoffe beigefügt wurden, zugemischt. Das Granulat wird zu Tabletten von 1.3 g verpresst.

### Beispiel 2:

200 Gewichtsteile Lactose pulv. werden auf 55°C erwärmt. In 45 Gewichtsteilen Ethanol löst man 10 Gewichtsteile Loratadin, anschliessend 10 Gewichtsteile Polyvinylpyrrolidon und 2.5 Gewichtsteile Aerosil^{(R)}. Diese Lösung wird unter Rühren auf die Lactose aufgebracht, dann wird 10 min zur gleichmässigen Verteilung gemischt. Anschliessend wird die Mischung mittels Vakuum bei 50°C getrocknet und auf 0.1 mm gesiebt. Das Wirkstoffgranulat wird zu 650 Gewichtsteilen einer Brausebasis zugefügt, auf die 0.2 Gewichtsteile Phosphatidylcholin aufgezogen wurden. Die Mischung wird weiters mit 2 Gewichtsteilen Zuckerester und 200 Gewichtsteilen eines Füllstoffes sowie Aromen und Süßstoffen versetzt und schliesslich zu Tabletten verpresst.

Eine Modifikation dieser Vorschrift besteht darin, das Phosphatidylcholin (z.B. Epikuron^{(R)}) gemeinsam mit dem Bindemittel und dem Wirkstoff auf den Träger aufzubringen. Die Suspensionswirkung ist dann allerdings nicht mehr ganz so gut.

Ein noch besseres Auflöseverhalten der Wirkstoffphase in der Brausetablette kann erzielt werden, wenn man geringe Mengen von Natriumbicarbonat in das Granulat einbringt, wodurch, da das Natriumbicarbonat beim Auflösen der Tablette mit der sauren Brauselösung reagiert, gröbere Teilchen noch zerfallen und den Wirkstoff in Suspension bringen.

### Beispiel 3:

10 Gewichtsteile Loratadin werden in 25 Gewichtsteilen Ethanol gelöst; anschliessend werden 10 Gewichtsteile Polyvinylpyrroldion gelöst sowie 5 Gewichtsteile Aerosil^{(R)} und 0.8 Gewichtsteile Natriumbicarbonat in die Lösung eingebracht, die auf 60°C erwärmt wird. Diese Lösung wird auf 100 Gewichtsteile Lactose unter Rühren aufgebracht und zur gleichmässigen Verteilung 5 min gemischt; anschließend wird getrocknet und auf 0.2 mm gesiebt. Dieses Wirkstoffgranulat wird mit 850 Gewichtsteilen Brausebasis sowie mit einer Mischung von 50 Gewichtsteilen Mannit und 2 Gewichtsteilen eines Zuckeresters von Speisefettsäuren, sowie mit Füll-, Süss- und Aromastoffen und 50 Gewichtsteilen einer Emulgator-Phase (bestehend aus 49.8 Gewichtsteilen Mannit und 0.2 Gewichtsteilen Phospholipide) vermischt und zu Tabletten verpresst.

### Beispiel 4:

5 Gewichtsteile Aerosil^{(R)}, 10 Gewichtsteile Loratadin und 100 Gewichtsteile Lactose D80 werden unter Mischen auf 50°C erwärmt. Darauf wird eine Lösung bestehend aus 10 Gewichtsteilen Polyvinylpyrrolidon und 37 Gewichtsteilen Ethanol, in der 0.8 Gewichtsteile Natriumbicarbonat suspendiert werden, in 3 Schritten auf die Mischung unter Rühren aufgebracht. Im 1. Schritt werden 55 bis 60 % der Lösung unter Rühren aufgebracht, 5 min verteilt, worauf das Lösungsmittel mittels Vakuum auf 100 mbar etwas abgetrocknet wird. Man bringt dann den 2. Teil der Lösung, ca. 42%, unter Rühren auf und mischt zur gleichmässigen Verteilung 2 min lang, wonach das Lösungsmittel wieder mittels Vakuum auf 100 mbar etwas abgetrocknet wird. Sodann wird der restliche Teil der Lösung unter Rühren aufgebracht und mittels Vakuum endgetrocknet.

Das so hergestellte Wirkstoffgranulat wird mit 50 Gewichtsteilen Emulgatorgranulat (bestehend aus 49.8 Teilen Mannit und 0.2 Gewichtsteilen Lecithin) sowie 850 Gewichtsteilen Brausebasis, Süss- und Aromastoff, sowie 100 Gewichtsteilen Sorbit und einer Mischung von 2.4 Gewichtsteilen Zuckerester einer Speisefettsäure mit 50 Gewichtsteilen Mannit, zu Brausetabletten von 1.25 g verpresst.

### Beispiel 5:

Bei gleicher Zusammensetzung wie Beispiel 4. kann z. B. auch eine Teilmenge von Aerosil^{(R)} in die Lösung eingebracht werden, sowie auch eine Teilmenge von Polyvinylpyrrolidon zur Mischung von Aerosil^{(R)} und Lactose trocken zugefügt werden. Diese Mischung wird sodann weiter wie im Beispiel 4 behandelt.

### Beispiel 6:

### Herstellung des Emulgatorgranulates:

50 Gewichtsteile Mannit werden unter Rühren auf 50°C erwärmt. Es wird eine Lösung aus 0.2 Gewichtsteilen von Epikuron^{(R)} in 12 Gewichtsteilen Ethanol bereitet und unter Rühren auf das Mannit aufgebracht. Anschliessend wird mittels Vakuum bei einer Produkttemperatur von 50°C getrocknet und das Granulat auf 0,3 mm gesiebt.

### Beispiel 7:

### Herstellung der Cisaprid Phase:

10 Gewichtsteile Cisaprid werden mit 10 Gewichtsteilen Aerosil^{(R)} vorgemischt, auf 40°C aufgewärmt und mit einer Ethanol-Aceton-Lösung (2:18) befeuchtet, die 1 Gewichtsteil Propylenglycol, 1 Gewichtsteil Docusat-Natrium und 2 Gewichtsteile Polyvinylpyrrolidon enthält. Die Lösung wird unter Rühren 5 min lang verteilt; anschliessend wird mittels Vakuum unter Rühren getrocknet. Das Wirkstoffgranulat wird auf 0,1 mm Korngrösse gesiebt.

### Herstellung des Brause-Granulates:

790 Gewichtsteile Zitronensäure, 16 Gewichtsteile Äpfelsäure sowie 9,5 Gewichtsteile Saccharin-Natrium werden unter Mischen auf 60°C erwärmt. Darauf werden 4,6 Gewichtsteile einer Lösung bestehend aus 0,5 Gewichtsteilen Natriumcitrat, 0,7 Gewichtsteilen Zitronensäure, 2,5 Gewichtsteilen Wasser, 0,9 Gewichtsteilen Sorbitol aufgebracht und 3 Minuten unter Mischen verteilt. Man fügt anschliessend 274 Gewichtsteile Natriumhydrogencarbonat sowie 2 Gewichtsteile Aspartam zu und lässt kurz anreagieren. Anschliessend werden 62 Gewichtsteile Natriumcarbonat zugemischt und das Produkt unter langsamen Rühren mittels Vakuum getrocknet. Das Produkt wird auf 1,6 mm Korngrösse gesiebt.

### Herstellung der Endmischung:

Zu der Wirkstoff-Phase sowie zu dem Brausegranulat werden unter Zusatz eines Anti-Schaummittels (0,1 Gewichtsteile Polysiloxan auf 50 Gewichtsteile Mannit aufgezogen) 2 Gewichtsteile Metarin P in Mischung mit 100 Gewichtsteilen Lactose sowie 50 Gewichtsteilen Maltodextrin, 150 Gewichtsteilen Mannit und 20 Gewichtsteilen Zitronenaroma gemischt und anschliessend zu Tabletten verpresst.

### Beispiel 8:

### Herstellung der Cisaprid-Phase:

270 Gewichtsteile Mannit und 5 Gewichtsteile Aerosil^{(R)} sowie 10 Gewichsteile Natriumbicarbonat werden unter Rühren gemischt und auf 60°C erwärmt. Sodann wird eine Lösung aus 2 Gewichtsteilen Polyvinylpyrrolidon, 0,8 Gewichtsteilen Docusat-Natrium, 1 Gewichtsteil Propylenglycol und 10 Gewichtsteile Cisaprid in 2 Gewichtsteilen Ethanol und 30 Gewichtsteilen 2-Butanon gelöst; diese Lösung wird in 2 Teilen auf die Träger gebracht, worauf eine Zwischentrocknung auf 100 mbar erfolgt, wonach das Granulat endgetrocknet und auf eine Korngrösse von 0,2 mm gesiebt wird.

### Herstellung des Brausegranulates:

Die Herstellung des Brausegranulates erfolgt wie unter Beispiel 7 angegeben.

### Herstellung des Emulgator-Granulates:

20 Gewichtsteile Mannitol werden auf 50°C erwärmt; sodann wird eine Lösung von 0,4 Gewichtsteilen Metarin F in 2 Gewichtsteilen Ethanol gelöst und unter Rühren auf das Mannitol aufgebracht. Anschliessend wird das Lösungsmittel mittels Vakuum weggetrocknet und das so hergestellte EmulgatorGranulat auf 0,3 mm gesiebt.

### Herstellung der Endmischung:

Die drei Phasen werden unter Zusatz eines Antischaummitels (0,1 Gewichtsteile Polysiloxan auf 50 Gewichtsteile Mannit aufgezogen), 50 Gewichtsteile Maltodextrin, sowie 40 Gewichtsteile Orangenaroma gemischt und anschliessend zu Tabletten mit 1,6 g verpresst.

## Patentansprüche

1. Pharmazeutische Zubereitung, enthaltend einen hydrophoben Wirkstoff, insbesondere Loratadin oder Cisaprid, ein Tensid und ein Brausesystem, **dadurch gekennzeichnet**, dass die Zubereitung wenigstens zwei verschiedene Tenside bzw. Emulgatoren enthält.

2. Zubereitung nach Anspruch 1, **dadurch gekennzeichnet**, dass wenigstens ein Tensid Docusat-Natrium oder Natriumlaurylsulfat, bzw. wenigstens ein Emulgator aus der Gruppe der Zuckerester, der Polysorbate und der Phospholipide gewählt ist.

3. Zubereitung nach Anspruch 1 oder 2, **dadurch gekennzeichnet**, dass wenigstens einer der Emulgatoren getrennt vom Brausesystem und/oder Wirkstoffgranulat in einem eigenen Granulat in der Mischung vorliegt.

4. Zubereitung nach einem der vorhergehenden Ansprüche, **dadurch gekennzeichnet**, dass der Wirkstoff in einem eigenen Wirkstoffgranulat - vorzugsweise mit Hilfe eines Bindemittels, wie z.B. Polyvinylpyrrolidon - auf der Oberfläche eines Schwebstoffes bzw. Antiklebstoffes, wie z. B. Aerosil^{(R)} oder Avicel^{(R)}, niedergeschlagen ist.

5. Zubereitung nach Anspruch 4, **dadurch gekennzeichnet**, dass das Wirkstoffgranulat ausserdem wenigstens eine der Brausekomponenten und/oder einen Füllstoff enthält, der vorzugsweise wenigstens eine der folgenden Verbindungen umfasst: höhere Alkohole, z.B. Mannit, Sorbit oder Lactose; Hydrokolloide, wie Maltodextrin oder Dextrine; Stärke.

6. Zubereitung nach einem der vorhergehenden Ansprüche, **dadurch gekennzeichnet**, dass wenigstens ein Emulgator auf den Komponenten des Brausesystems bzw. einem eine Brausekomponente enthaltenden Granulat und/oder auf dem Wirkstoffgranulat niedergeschlagen ist.

7. Verfahren zur Herstellung eines Wirkstoffgranulates für eine Zubereitung nach einem der Ansprüche 3 bis 6, **dadurch gekennzeichnet**, dass eine Lösung, die den Wirkstoff - sowie vorzugsweise wenigstens ein Bindemittel und/oder wenigstens einen Emulgator - enthält, auf die Schwebstoff- und/oder Füllstoffpartikel aufgetragen, gleichmässig verteilt und erwärmt wird, worauf das Lösungsmittel - vorzugsweise im Vakuum - abgetrocknet und das entstandene Granulat auf die gewünschte Korngrösse gesiebt wird.

8. Verfahren zur Herstellung eines Wirkstoffgranulates für eine Zubereitung nach einem der Ansprüche 3 bis 6, **dadurch gekennzeichnet**, dass der Wirkstoff in Mischung mit dem Schwebstoff und/oder Füllstoff mit einer Lösung benetzt und auf den Partikeln gleichmässig verteilt wird, welche Lösung wenigstens ein Bindemittel und/oder wenigstens einen Emulgator enthält, worauf unter Rühren granuliert, - vorzugsweise im Vakuum - getrocknet und auf die gewünschte Korngrösse gesiebt wird.

9. Verfahren zur Herstellung eines Emulgatorgranulates für eine Zubereitung nach einem der Ansprüche 3 bis 6, **dadurch gekennzeichnet**, dass eine Lösung, die wenigstens einen Emulgator - und gegebenenfalls wenigstens ein Bindemittel - enthält, auf den Füllstoff aufgezogen wird, worauf unter Rühren granuliert, - vorzugsweise im Vakuum - getrocknet und auf die gewünschte Korngrösse gesiebt wird.

10. Verfahren nach einem der Ansprüche 7 bis 9, **dadurch gekennzeichnet**, dass der Füllstoff wenigstens eine der folgenden Verbindungen umfasst: höhere Alkohole, z.B. Mannit, Sorbit oder Lactose; Hydrokolloide, wie Maltodextrin oder Dextrine; Stärke,

## Claims

1. Pharmaceutical formulation, containing a hydrophobic active substance, in particular loratadin or cisapride, a surfactant and an effervescent system, **characterized in that** the formulation contains at least two different surfactants or emulsifiers.

2. Formulation according to Claim 1, **characterized in that** at least one surfactant, docusate sodium or sodium laurylsulphate, or at least one emulsifier from the group consisting of the sugar esters, of the polysorbates and of the phospholipids is chosen.

3. Formulation according to Claim 1 or 2, **characterized in that** at least one of the emulsifiers is present as separate granules in the mixture, separated from the effervescent system and/or granules of active substance.

4. Formulation according to any of the preceding Claims, **characterized in that** the active substance, in separate granules of active substance, is deposited on the surface of a suspended substance or antiadhesive, such as, for example, Aerosil^{(R)} or Avicel^{(R)}, preferably with the aid of a binder, such as, for example, polyvinylpyrrolidone.

5. Formulation according to Claim 4, **characterized in that** the granules of active substance additionally contain at least one of the effervescent components and/or a filler, which preferably comprises at least one of the following compounds: higher alcohols, e.g. mannitol, sorbitol or lactose; hydrocolloids, such as maltodextrin or dextrins; starch.

6. Formulation according to any of the preceding Claims, **characterized in that** at least one emulsifier is applied to the components of the effervescent system or to granules containing an effervescent component and/or to the granules of active substance.

7. Process for the preparation of granules of active substance for a formulation according to any of Claims 3 to 6, **characterized in that** a solution which contains the active substance and preferably at least one binder and/or at least one emulsifier is applied to the particles of suspended substance and/or filler particles, uniformly distributed and heated, after which the solvent is removed by drying, preferably under vacuum, and the resulting granules are sieved to the desired particle size.

8. Process for the preparation of granules of active substance for a formulation according to any of Claims 3 to 6, **characterized in that** the active substance mixed with the suspended substance and/or filler is wet with a solution and is uniformly distributed over the particles, which solution contains at least one binder and/or at least one emulsifier, after which granulation is carried out while stirring, drying is carried out, preferably under vacuum, and sieving is carried out to the desired particle size.

9. Process for the preparation of emulsifier granules for a formulation according to any of Claims 3 to 6, **characterized in that** a solution which contains at least one emulsifier and optionally at least one binder is applied to the filler, after which granulation is carried out while stirring, drying is carried out, preferably under vacuum, and sieving is carried out to the desired particle size.

10. Process according to any of Claims 7 to 9, **characterized in that** the filler comprises at least one of the following compounds: higher alcohols, e.g. mannitol, sorbitol or lactose; hydrocolloids, such as maltodextrin or dextrins; starch.

## Revendications

1. Préparation pharmaceutique contenant un principe actif hydrophobe, en particulier la loratadine ou le cisapride, un tensioactif et un système effervescent, **caractérisée en ce qu**'elle contient au moins deux agents tensioactifs ou émulsifiants différents.

2. Préparation selon la revendication 1, **caractérisée en ce qu**'elle contient au moins un tensioactif choisi parmi le docusate de sodium et le laurylsulfate de sodium ou au moins un émulsifiant choisi dans le groupe des esters des sucres, des polysorbates et des phospholipides.

3. Préparation selon la revendication 1 ou la revendication 2, **caractérisée en ce qu**'au moins un des émulsifiants est présent dans le mélange, dans des granulés spécifiques distincts du système effervescent et / ou des granulés du principe actif.

4. Préparation selon l'une des revendications précédentes, **caractérisée en ce que** le principe actif dans des granulés spécifiques est appliqué - de préférence avec l'aide d'un liant tel que, par exemple, la polyvinylpyrrolidone - sur la surface d'un agent dispersant ou antiagglomérant tel que, par exemple, le produit Aerosil ® ou Avicel ®.

5. Préparation selon la revendication 4, **caractérisée en ce que** les granulés du principe actif contiennent, en outre, au moins un des composants du système effervescent et / ou un excipient comprenant, de préférence, au moins un des composés suivants : alcools supérieurs, par exemple le mannitol, le sorbitol ou le lactose ; hydrocolloïdes comme la maltodextrine ou la dextrine ; amidons.

6. Préparation selon l'une des revendications précédentes, **caractérisée en ce qu**'au moins un émulsifiant est déposé sur les composants du système effervescent ou sur les granulés contenant un composé du système effervescent et / ou sur les granulés du principe actif.

7. Procédé pour préparer des granulés d'un principe actif destinés à une préparation selon l'une des revendications 3 à 6, **caractérisé en ce qu**'une solution, qui contient le principe actif ainsi que, de préférence, au moins un agent liant et / ou au moins un émulsifiant, est appliquée et répartie de manière uniforme sur les particules du dispersant et / ou de l'excipient, ceci étant suivi par un chauffage, effectué de préférence sous vide, en vue de l'évaporation du solvant et par un tamisage des granulés résultants pour avoir la granulométrie souhaitée.

8. Procédé pour préparer des granulés d'un principe actif destinés à une préparation selon l'une des revendications 3 à 6, **caractérisé en ce que** le principe actif en mélange avec le dispersant et / ou l'excipient est humecté sous agitation avec une solution qui est répartie de manière uniforme sur les particules et qui contient au moins un agent liant et / ou au moins un émulsifiant, en vue d'opérer une granulation, qui est suivie d'un séchage - effectué de préférence sous vide - et d'un tamisage pour avoir la granulométrie souhaitée.

9. Procédé pour préparer des granulés d'un émulsifiant destinés à une préparation selon l'une des revendications 3 à 6, **caractérisé en ce qu**'une solution, qui contient au moins un émulsifiant et, le cas échéant, au moins un agent liant, est appliquée sous agitation sur l'excipient, en vue d'opérer une granulation, qui est suivie d'un séchage - effectué de préférence sous vide - et d'un tamisage pour avoir la granulométrie souhaitée.

10. Procédé selon l'une des revendications 7 à 9, **caractérisé en ce que** l'excipient comprend au moins un des composés suivants : alcools supérieurs, par exemple le mannitol, le sorbitol ou le lactose ; hydrocolloïdes, comme la maltodextrine ou la dextrine ; amidons.
